# EUROPEAN PATENT APPLICATION

(11) **EP 2 248 824 A1**
(43) Date of publication of application: **10.11.2010**
(21) Application number: 09005849.6
(22) Date of filing: 27.04.2009
(51) Int. Cl.: C07K 14/435, C12P 1/00, C12P 3/00

(54) **Use of silintaphin for the structure-directed fabrication of (nano)composite materials in medicine and (nano) technology**

(71) Applicant: Wiens,, Matthias, Dr., 55270 Essenheim (DE); MÜLLER, Werner E. G., 65203 Wiesbaden (DE); Schröder, Heinz C., 65189 Wiesbaden (DE); Wang,, Xiaohong, Prof. Dr., Beijing 100875 (CN)
(72) Inventor: Wiens,, Matthias, Dr., 55270 Essenheim (DE); MÜLLER, Werner E. G., 65203 Wiesbaden (DE); Schröder, Heinz C., 65189 Wiesbaden (DE); Wang,, Xiaohong, Prof. Dr., Beijing 100875 (CN)
(74) Representative: Krauss, Jan

(57) **Abstract**

The invention concerns the application of silintaphin-1 in the sustainable fabrication of hierarchically ordered silica structures from nano- to macro-scale at environmentally benign conditions and low energy costs (low temperature, low pressure, absence of caustic chemicals).

## Description

The present invention relates to the application of silintaphin-1 in the sustainable fabrication of hierarchically ordered silica structures from nano- to macroscale at environmentally benign conditions and low energy costs (low temperature, low pressure, absence of caustic chemicals).

### Background of Invention

The present invention relates to a novel technology that allows the biomimetic synthesis of silica, a major material used in nanotechnology, including the fabrication of opto- and microelectronics. The technology is based on two unique proteins, silicatein and silintaphin-1.

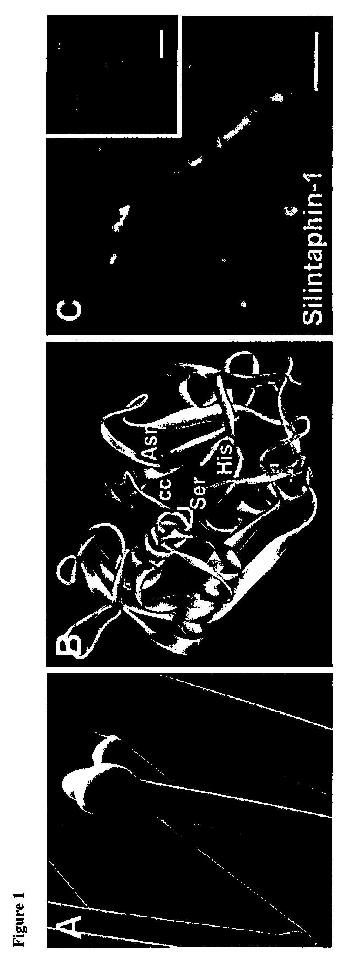

Both proteins have been isolated and cloned from sponges. They are present in the micro- and macroscale spicules (skeletal elements) of siliceous sponges (Figure 1A) and are available as recombinant proteins.

Silicatein of siliceous sponges (Figure 1B) is able to catalyze the formation of silica ("biosilica") following an enzymatic process. Biosilica is the inorganic component of the sponge spicules. The state-of-the-art in the morphology and the biogenesis of spicules has been described in: Uriz et al. (2003) Progr Molec Subcell Biol 33:163-193; Müller et al. (2003) Progr Molec Subcell Biol 33:195-221. Accordingly, silicatein is at the crossroads between the inorganic and the organic (living) world. Several isoforms have been identified so far.

Recombinant silicatein can be immobilized on various surfaces (metal, metal oxide, silicon wafers, etc.) without loss of biological activity. The immobilized enzyme can be used for biocatalytic formation of nanolayers of silica (and other metal oxides such as titanium oxide and zirconium oxide) under mild conditions (low temperature and near-neutral pH) for application in medicine, dentistry and microelectronics (applying soft lithography procedures).

Natural biosilica structures (for example: sponge spicules) are characterized by a genetically determined and defined morphology. It turned out that silicatein alone is not able to direct the size and shape of 3D silica structures. Now a second breakthrough protein has been discovered that (1) has structure-directing activity and (2) allows extension of the range of applications of silicatein from nano-/micro-scale to macro-scale.

The unique abilities of these two proteins (silicatein and silintaphin-1) render their concerted action highly interesting for (nano)biotechnological and biomedical applications. Hitherto used methods for the production of silica (glass) require the presence of high temperature, pressure, and aggressive chemicals. In contrast sponges are able to synthesize siliceous nanostructures enzymatically (biocatalysis) under biological and environmentally benign conditions with great precision and reproducibility.

The present invention relates to a novel technology, representing a significant progress beyond the state-of-the-art, which is based on the application of the novel silicatein-binding protein silintaphin-1, based on surprising properties of this protein as found by the inventors.

Silintaphin-1 forms the "core" of the natural silicatein filaments ("axial filaments") of the sponge spicules (Figure 1C). It directs the assembly of silicatein units. The inventors demonstrate that it is now for the first time possible to synthesize "biosiliceous" structures using silintaphin-1, which may be of interest for a great variety of biotechnological/biomedical applications.

Silicatein (silicatein-α) and the application of this enzyme in various areas of technology and biomedicine have been patented by the inventors (German Patent No. DE10037270, European Patent No. EP1320624, United States Patent No. US 7,169,589 B2, Chinese Patent No. ZL 01813484.X, New Zealand Patent No. 523474, Australia Patent No. 2001289713. Silicatein-mediated synthesis of amorphous silicates and siloxanes and their uses. Inventors: Müller WEG, Lorenz A, Krasko A, Schröder HC; national phases: NO20030407; Japan No. 2002-516336; Canada No. 2,414,602). Review articles: W.E.G. Müller, X. Wang, S.I. Belikov, W. Tremel, U. Schloßmacher, A. Natoli, D. Brandt, A. Boreiko, M.N. Tahir, I.M. Müller and H.C. Schröder: Formation of siliceous spicules in demosponges: example Suberites domuncula. In Handbook of Biomineralization. Vol. 1: Biological Aspects and Structure Formation (ed. by E. Bäuerlein). Wiley-VCH, Weinheim, pp. 59-82 (2007); H.C. Schröder, D. Brandt, U. Schloßmacher, X. Wang, M.N. Tahir, W. Tremel, S.I. Belikov and W.E.G. Müller: Enzymatic production of biosilica glass using enzymes from sponges: basic aspects and application in nanobiotechnology (material sciences and medicine). Naturwissenschaften 94, 339-359 (2007); H.C. Schröder, X. Wang, W. Tremel, H. Ushijima and W.E.G. Müller: Biofabrication of biosilica-glass by living organisms. Nat. Prod. Rep. 25, 455-474 (2008).

Besides silicatein-α (patents see above), further silicateins were cloned by the inventors, including silicatein-β (patent application: DE10352433.9. Enzym- und Template-gesteuerte Synthese von Silica aus nicht-organischen Siliciumverbindungen sowie Aminosilanen und Silazanen und Verwendung. Inventors: Schwertner H, Müller WEG, Schröder HC), and four silicatein isoforms from a freshwater sponge (silicatein-a1-4; DE102006001759.5. Kontrollierte Herstellung von Silber- und Gold-Nanopartikeln und Nanokristallen definierter Größe und Form durch chirale Induktion mittels Silicatein. Inventors: Tremel W, Tahir MN, Müller WEG. Schröder HC). In addition, silicatein from another marine sponge (*Tethya lyncurium*; PCT/US99/30601. Methods, compositions, and biomimetic catalysts, such as silicateins and block copolypeptides, used to catalyze and spatially direct the polycondensation of silicon alkoxides, metal alkoxides, and their organic conjugates to make silica, polysiloxanes, polymetallo-oxanes, and mixed poly(silicon/metallo)oxane materials under environmentally benign conditions. Inventors: Morse DE, Stucky GD, Deming, TD, Cha J, Shimizu K, Zhou Y) has been patented.

Another enzyme involved in silica metabolism is the silicase which belongs to the group of carbonic anhydrases (German Patent No. DE10246186. In vitro and in vivo degradation or synthesis of silicon dioxide and silicones, useful e.g. for treating silicosis or to prepare prosthetic materials, using a new silicase enzyme. Inventors: Müller WEG, Krasko A, Schröder HC). This enzyme, which has first been discovered in the marine sponge *S. domuncula*, is able to dissolve silica under formation of free silicic acid (Schröder et al. (2003) Progr Molec Subcell Biol 33:250-268). In addition, the silicase - in the reverse reaction - may also mediate the synthesis of the silica polymer (DE10352433.9. Enzymatische Synthese, Modifikation und Abbau von Silicium(IV)- und anderer Metall(IV)-Verbindungen. German Patent Office 2003. Inventors: Müller WEG, Schwertner H, Schröder HC).

Silintaphin-1 is a novel silicatein-binding protein which directs the assembly of silicatein filaments. Natural biosilica structures (for example: sponge spicules) are characterized by a defined morphology. It turned out that silicatein alone is not able to direct the size and shape of 3D silica structures. The new silicatein interactor protein, silintaphin-1 (silicatein-α interactor with PH domain-1) was identified using the yeast two-hybrid system with a silicatein-α bait (Wiens M, Bausen M, Natalio F, Link T, Schlossmacher U, Müller WEG: The role of the silicatein-alpha interactor silintaphin-1 in biomimetic biomineralization. Biomaterials. 30: 1648-1656, 2009). Silintaphin-1 comprises a pleckstrin homology domain (PH domain) which is framed by eight C-terminal repeats of 10/12 amino acids and eight N-terminal repeats of 4-5 amino acids. The silintaphin-1 PH domain is required for the binding of silicatein. The entire silintaphin-1 sequence shows no homologies to any other known protein. Silintaphin-1 forms the "core" of the natural silicatein filaments ("axial filaments") of the sponge spicules (Figure 1 C). The inventors demonstrate that it is now for the first time possible to synthesize "biosilica" and other metal oxide structures, which may be of interest for a great variety of biotechnological/biomedical applications, from the nano- to macroscale.

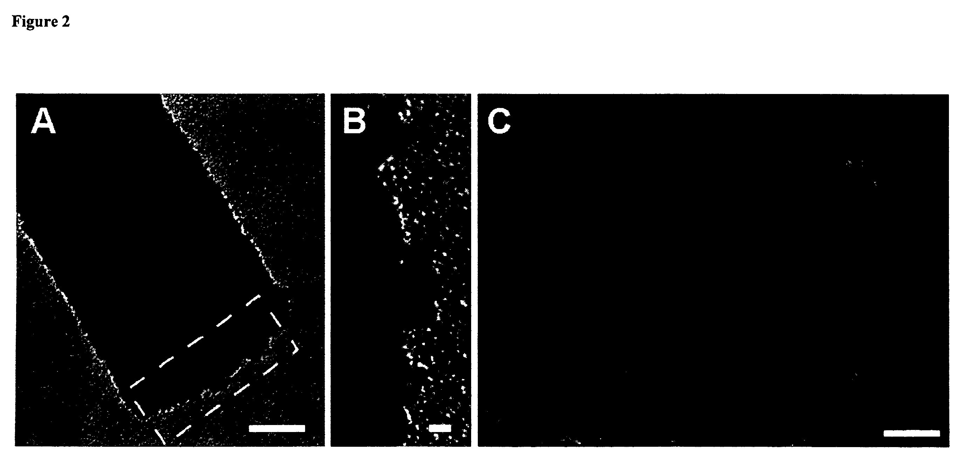

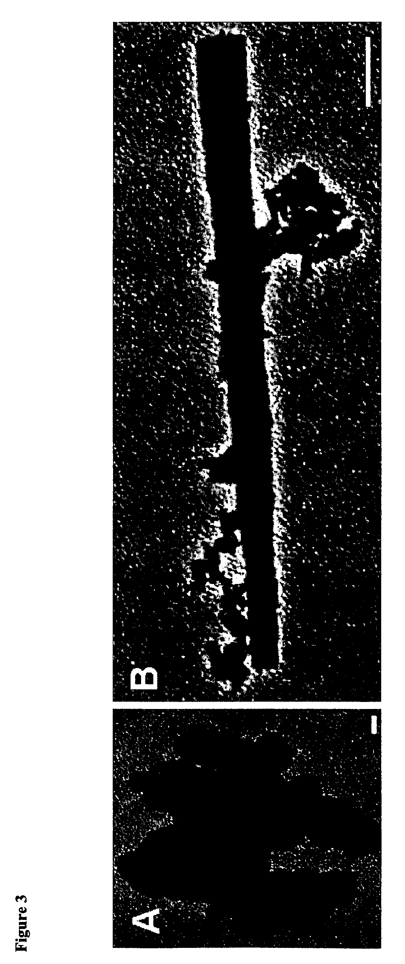

The discovery of silintaphin-1 was a breakthrough. The recombinant protein has a structure-directing activity: it interacts with recombinant silicatein that had been immobilized, e.g., on functionalized γ-Fe₂O₃ nanoparticles (Figure 2 A,B) or surfaces of silicon wafers (Figure 2C); these structures are not formed by the single proteins (Figure 3A). Using a combination of both proteins as template, structured composite materials can be generated, e.g., with rod-like morphology (Figure 3B).

One aspect of this invention is the unexpected property of nanorods, nanowires, and nanobullets thus produced by application of silintaphin-1 and metal oxide nanoparticles: The inventors demonstrate that the structures obtained can be used as optical waveguides. The light can be produced (i) by an external source and then coupled into the structures or (ii) internally by incorporation of fluorescent proteins (e.g., GFP, RFP), light producing enzymes (luciferase, alkaline phosphatase) and their substrates (luciferin, disodium 2-chloro-5-(4-methoxyspiro{1,2-dioxetane-3,2'-(5'-chloro)-tricyclo[3.3.1.1.3,7]decan}-4-yl)phenyl phosphate), or fluorophores into the structures.

Moreover, structures combining this technology with the silica encapsulation of bacterial cells may provide novel sensors for a variety of applications.

### Biomimetic optical fibres (waveguides)

The combined application of silicatein and silintaphin-1 allows the synthesis of biomimetic optical fibres that show unexpected, advantageous properties compared to industrial optical fibres: extreme stability, formation under environmental benign and low energy-cost conditions, transmission of selected wavelengths. Using the combined action of silicatein and silintaphin, the formation of waveguides (optical fibres; imitating the model found in nature; Figure 3) is now feasible even in the macroscale.

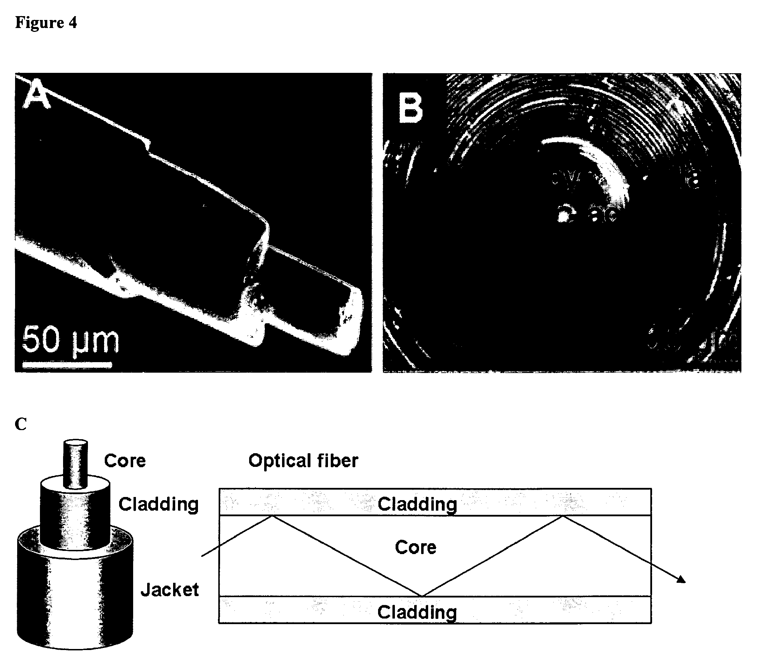

Optical fibres used in telecommunication consist of a core and a lower-refractive-index cladding, which are made of silica, and a protective outer coating (Figure 4C left). Light is transmitted in the core by total internal reflection (Figure 4C right). Such optical fibres show a striking similarity to spicules of siliceous sponges, in particular spicules of the hexactinellid sponges (glass sponges) (Figure 4A,B). Spicules of these sponges can reach a length of up to 3 meters and a diameter of up to 8.5 mm; thus they are the largest biosilica structures on earth. Hexactinellid spicules are composed of up to 600 silica lamellae, surrounding an axial canal which harbours the proteinaceous axial filament (Müller et al. Cell Tissue Res. 329, 363, 2007). Sponge spicules act as optical glass fibers, which transmit light with high efficiency (Cattaneo-Vietti et al. Nature 383, 397, 1996; Aizenberg et al. Proc. Natl. Acad. Sci. USA 101, 3358, 2004; Müller et al. Biosens. Bioelectron. 21, 1149, 2006). They have a high refractive index core, a low refractive index surrounding cylindrical tube, and an outer portion with a progressively increasing refractive index (Sundar et al. Nature 424, 899, 2003). Spicules exhibit advantageous properties, compared to technical optical fibres, based on their composite structure and their lamellar architecture: enhanced fracture toughness, low-temperature synthesis, and presence of dopants (sodium), raising the refractive index. Spicules act as sharp high- and low pass filters; only wavelengths between 615 and 1310 nm can pass; wavelengths <615 nm and >1310 nm are filtered out (Müller et al. Biosens. Bioelectron. 21, 1149, 2006).

The discovery of silintaphin allows the formation of biomimetic optical fibers with extreme mechanical stability (due to the composite material). This property is not shown by technical optical fibers, and therefore this invention represents a significant progress beyond the state-of-the-art.

The possibility to direct the assembly of silica nanoparticles by the combined action of silicatein and silintaphin-1 also facilitates novel synthetic strategies to synthesize microstructured fibres. Moreover, structures combining this technology and the technique for encapsulation of bacterial cells (or biomolecules) allow the construction of sensors for a variety of technical applications.

### Biocatalytic encapsulation technology.

These applications can be combined with other techniques, such as encasing of bacterial sensors or biomolecules in a silica shell. We developed a technology for encapsulation of bacterial cells in a silica shell under mild conditions (e.g., absence of aggressive and caustic chemicals that may damage the cells). Bacteria are transformed with the silicatein gene. Silicatein expressed on the surface of the bacteria is then used for biocatalytic formation of a silica shell. This shell does not impair cell growth. Thus, encapsulated bacteria can be used as nanofactories or sensors in food technology, drug development, environmental monitoring, or medicine. Basic publication describing the technology for encapsulation of bacterial cells: W.E.G. Müller, S. Engel, X. Wang, S.E. Wolf, W. Tremel, N.L. Thakur, A. Krasko, M. Divekar and H.C. Schröder: Bioencapsulation of living bacteria (Escherichia coli) with poly(silicate) after transformation with silicatein-α gene. Biomaterials 29, 771-779 (2008).

### Summary of Invention

This invention relates to the unexpected waveguiding properties of nanorods, nanowires, and nanobullets formed by self-assembly of silica and other metal oxides (nanoparticulate or soluble enzyme substrates) using either silintaphin-1 alone or silintaphin-1 and silicatein. Silintaphin-1 and silicatein are two proteins with unique properties: *In vivo* they act in concert in the assembly of nanoscale silica particles (nanospheres) to skeletal structures. The opto-mechanical properties of the resulting nanocomposite waveguides, consisting of both a proteinaceous and an inorganic component, are superior to those consisting of the inorganic component alone. Thus, the nanorods and nanowires formed can be used as novel optical fiber-based bacterial sensors and evanescent wave sensors that can be fabricated at mild (low temperature and near neutral pH) conditions.

### Detailed Description of invention

In one preferred aspect thereof, the present invention relates to a method for the fabrication of nanorods/nanowires/nanobullets from metal oxide particles, wherein a polypeptide is used for the synthesis, **characterized in that** the polypeptide comprises an animal, bacterial, plant or fungal silintaphin-1, in particular the PH domain thereof, that exhibits a sequence similarity of at least 25%, preferably a sequence percent identity, to the amino acid sequence shown in SEQ ID No. 1. Further preferred is a percent similarity of at least 50%, more preferred of at least 75%. Even more preferred is a percent identity of said domain of at least 50%, more preferred of at least 75%.

Percent similarities and identities can be determined using the algorithms as available in the state of the art and as known by the person of skill, such as, for example, the program ClustalW.

More preferred is a method according to the present invention, wherein in addition to metal oxide particles silintaphin-1 and Glu-tagged silicatein-coated CaCO₃ (nano)particles, or silicatein and Glu-tagged silintaphin-1-coated CaCO₃ (nano)particles, or silicatein-coated silica nanoparticles and Glu-tagged silintaphin-1-coated CaCO₃ (nano)particles, or silicatein-coated nanoparticles of other metal oxides and Glu-tagged silintaphin-1-coated CaCO₃ (nano)particles are used for the fabrication of nanorods/nanowires/nanobullets. Preferred is a method, wherein instead of CaCO₃ other metal- or alkaline-earth metal carbonates are used for generation of nanorods/nanowires/nanobullets.

More preferred is a method according to the present invention, **characterized in that** the metal oxide particles used for synthesis are coated with a silintaphin-1-interacting molecule.

More preferred is a method according to the present invention, **characterized in that** the metal oxide particles used for the fabrication of the nanorods/nanowires/nanobullets consist of silica, titania, zirconia, or supermagnetic iron oxide.

Even more preferred is a method according to the present invention, wherein the silintaphin-1-interacting molecule is selected from at least one of a metal oxide forming protein, silicatein, and silicatein from the sponge *Suberites domuncula*.

Even more preferred is a method according to the present invention, wherein a mixture of two or several metal oxides is used.

More preferred is a method according to the present invention, wherein a silintaphin-1 polypeptide from *Suberites domuncula* according to SEQ ID No. 1 is used, or a polypeptide being homologous thereto, which in the amino acid sequence of the silintaphin-1 domain exhibits a sequence similarity, preferably identity, of at least 25% to the sequence shown in SEQ ID No. 1, or functional parts thereof. Further preferred is a percent similarity of at least 50%, more preferred of at least 75%. Even more preferred is a percent identity of said domain of at least 50%, more preferred of at least 75%.

More preferred is a method according to the present invention, wherein a silintaphin-1 polypeptide from *Suberites domuncula* according to SEQ ID No. 1 is used, or a polypeptide being homologous thereto, which in the amino acid sequence of the silintaphin-1 domain exhibits a sequence similarity, preferably identity, of at least 25% to the sequence shown in SEQ ID No. 1, is provided *in vivo,* in a cellular extract or lysate or in purified form. Further preferred is a percent similarity of at least 50%, more preferred of at least 75%. Even more preferred is a percent identity of said domain of at least 50%, more preferred of at least 75%.

More preferred is a method according to the present invention, wherein silicic acid, silicates, monoalkoxysilanetriols, monoalkoxysilanediols, monoalkoxysilanols, dialkoxysilane-diols, dialkoxysilanols, trialkoxysilanols, tetraalkoxysilanes, alkyl-, aryl- or metallo-silanetriols, alkyl-, aryl- or metallo-silanediols, alkyl-, aryl- or metallo-silanols, alkyl-, aryl- or metallomonoalkoxysilanediols, alkyl-, aryl- or metallo-monoalkoxysilanols, alkyl-, aryl- or metallodialkoxysilanols, alkyl-, aryl- or metallo-trialkoxysilanes or other metal oxide precursor compounds are used as substrates for synthesis.

More preferred is a method according to the present invention, wherein mixed polymers of defined composition are produced using defined mixtures of the compounds.

More preferred is a method according to the present invention, wherein the protein component is further used for facilitating densification of the material ("biosintering") by fusion of the silica particles or metal oxide particles.

More preferred is a method according to the present invention, wherein the nanorods/nanowires/nanobullets are further coupled to calcite microlenses (mimicking calcite microlenses of brittlestars and trilobites).

In another preferred aspect thereof, the present invention then relates to nanorods/nanowires/nanobullets produced according to a method according to the present invention.

In another preferred aspect thereof, the present invention then relates to a polypeptide of a silintaphin-1 from *Suberites domuncula* according to SEQ ID No. 1 or a polypeptide being homologous thereto, preferably according to any of SEQ ID Nos. 6 to 10, which in the amino acid sequence of the silintaphin-1 domain exhibits a sequence similarity, preferably identity, of at least 25% to the sequence shown in SEQ ID No. 1, or functional parts thereof. Further preferred is a percent similarity of at least 50%, more preferred of at least 75%. Even more preferred is a percent identity of said domain of at least 50%, more preferred of at least 75%.

More preferred is a nucleic acid according to the present invention according to SEQ ID No. 2, **characterized in that** it essentially, and preferably exclusively, encodes a polypeptide according to the present invention. Even further preferred is a nucleic acid according to the present invention, wherein said nucleic acid is a DNA, cDNA, RNA, or a mixture thereof. Preferably, wherein the sequence of said nucleic acid comprises at least one intron and/or a polyA sequence. The nucleic acid can be present in the form of its complementary "antisense" sequence.

In another preferred aspect thereof, the present invention then relates to a nucleic acid according to the present invention in the form (encoding for) of a (a) fusion protein (chimeric protein) construct or (b) construct with separate protein domains (separated by a protease cleavage site).

The nucleic acids according to the present invention can be produced synthetically.

In another preferred aspect thereof, the present invention then relates to a vector, preferably in form of a plasmid, shuttle vector, phagemid, cosmid, expression vector, retroviral vector, adenoviral vector or particle, nanoparticle or liposome, comprising a nucleic acid according to the present invention. More preferred is a vector, preferably in form of a nanoparticle or liposome, comprising a polypeptide according to the present invention.

In another preferred aspect thereof, the present invention then relates to a host cell transfected with a vector or infected or transduced with a particle according to the present invention. Further preferred is a host cell according to the present invention, wherein a polypeptide according to the present invention or parts thereof are expressed.

The polypeptide according to the present invention can be produced synthetically.

The polypeptide according to the present invention can be furthermore present in a prokaryotic or eukaryotic cell extract or lysate. Preferably, said polypeptide according to the present invention is used and present purified and essentially free from other proteins.

In another preferred aspect thereof, the present invention then relates to a nanocomposite material obtained using a method according to the present invention. Preferably suitable additives and supplements can be present together wit said nanocomposite material comprising a compound produced according to the present invention.

In another preferred aspect thereof, the present invention then relates to a use of the nanorods/nanowires generated according to the present invention as optical waveguides, as optical fibre-based evanescent wave sensors, as optical-fibre based bacterial sensor, as photonic crystals, as photonic crystal fibres and/or as light-emitting diodes (LEDs).

Preferred is a use according to the present invention as optical waveguides, wherein specific antibodies against selected molecules can be immobilized on the nanorods/nanowires/nanobullets surface as well as hydroxyapatite (HA) nanoparticles or nanoparticles consisting of CaCO₃ or other metal- or alkaline-earth metal carbonates due to the Glu-tag of recombinant silicatein or silintaphin-1.

### Application of nanorods/nanowires/nanobullets as optical waveguides

The recombinant proteins (silintaphin-1, various isoforms of biocatalytically active silicatein, luciferase, and GFP) required for the formation of nanorods/-wires/-bullets can be expressed in a recombinant way, e.g., by *Escherichia coli* using the Gateway-Technology and pDEST17 vector. cDNAs encoding various silicatein isoforms (e.g., silicatein-α and -β of the marine sponge *S. domuncula* and silicatein-α1 to silicatein-α4 of the freshwater sponge *Lubomirskia baicalensis*) have been used. The recombinant oligohistidine-tagged proteins can be purified, e.g., by Ni-NTA affinity chromatography. In addition, based on Glu-rich sequences found in several mammalian proteins that confer high binding affinity to hydroxyapatite (HA; Ca₅(PO₄)₃(OH)) of bone and tooth a novel protein-tag (Glu-tag) was developed by the inventors. Thus, Glu-tagged proteins can not only be purified by HA affinity chromatograph but also they can be immobilized on synthetic or natural CaCO₃ (nano)particles (bone, tooth, aragonite, calcite, vaterite), crystalline or amorphous.

Self-assembled nanorods, nanowires, nanobullets, and other structures of defined geometry can be generated in solution containing either:
- silintaphin-1 and silica nanoparticles, or
- silintaphin-1 and silicatein-coated silica nanoparticles, or
- silintaphin-1 and silicatein-coated nanoparticles of other metal oxides, or
- silintaphin-1, silicatein, and soluble silicatein subtrates (e.g., tetraethoxysilane (TEOS)) that are enzymatically processed to nanoparticulate products, or
- silintaphin-1 and silicatein-coated CaCO₃ (nano)particles, or
- silicatein and silintaphin-1-coated CaCO₃ (nano)particles, or
- silicatein-coated silica nanoparticles and silintaphin-1-coated CaCO₃ (nano)particles, or
- silicatein-coated nanoparticles of other metal oxides and silintaphin-1-coated CaCO₃ (nano)particles

These structures can also be generated from other metal- or alkaline-earth metal carbonates instead of calcium carbonate.

Silica nanoparticles of various sizes can be used (for example - preferred diameter: 10-20 nm). Various isoforms of silicatein and various combinations of isoforms can be used. Different state-of-the-art techniques for immobilization of silicatein to the nanoparticles (silica nanoparticles, magnetic γ-Fe₂O₃ [maghemite] nanoparticles, etc.) can be applied; e.g., binding via a reactive polymer containing catechol groups (binding to metal oxide surfaces) and nitrilotriacetic acid (NTA) groups (binding to the His-tagged recombinant proteins).

Based on this mechanism, silica nanorods, nanowires, and nanobullets of various diameters can be synthesized. Structures with diameters <1 µm act as single-mode (or few-mode) waveguides (in air or water), while diameters >1 µm allow multi-mode waveguiding of visible and infrared light.

In addition, the nanorods/nanowire/nanobullet light waveguides can be coupled to calcite microlenses (mimicking calcite microlenses of brittlestars and trilobites). The resulting hybrid bio-optical micro-devices efficiently guide, collect, and concentrate light.

A further aspect of this invention is that the formation of nanorods, nanowires, and nanobullets at ambient conditions allows the inclusion of dopants (e.g., sodium), which cannot be used in technical fabrication processes of optical fibres at high temperatures due to devitrification, to raise the refractive index of the structures generated. The formation of nanorods, nanowires, and nanobullets at ambient conditions according to the method described by the inventors also allows the entrapment of inorganic or organic dye molecules which would be decomposed at the higher temperatures used in technical fabrication processes.

In the absence of silica, silintaphin-1 and silicatein are able to form filaments consisting of one or both protein species (Figure 2C). In the presence of silica nanoparticles, rod-, wire-, and bullet-like shapes are formed. Thereby, unexpectedly, a hardening of the silica structures directed by silintaphin-1 occurs. In the presence of silintaphin-1 molecules carrying cell wall anchor sequence, pili-like rods/wires/bullets linked to individual bacteria can be obtained. In this way, light generated by bioluminescent or fluorescent bacteria can be effectively coupled into the attached waveguide, allowing the monitoring of signals emitted by individual bacteria.

It is also possible to generate nanorods/nanowires/nanobullets surrounded by several layers of silica lamellae (or lamellae of other metal oxides) based on molecular self-assembly of silintaphin-1 and silicatein on the surface of preformed, artificial nanorods, nanowires, and nanobullets. Enzymatic silica deposition under ambient conditions (via silicatein) can be performed in the presence of TEOS as substrate. This procedure can be repeated several times to obtain silica layers of decreasing or increasing refractive indexes by addition of dopants (e.g., decreasing or increasing amounts of sodium) in consecutive cycles of the fabrication procedure of the nanorods/nanowires/nanobullets at ambient conditions. The thickness of the layers (lamellae) can be modulated by varying the concentration or molar ratio of silinthaphin-1 and silicatein, the concentration of TEOS, or the incubation period. Moreover, the reaction can be performed in the presence of silica nanoparticles (diameter, 5-50 nm) to increase the thickness of the silica layers generated. Doping of the stepwise synthesized silica layers (lamellae) with decreasing amounts of sodium (at increasing distance from the optical axis) allows the generation of multi-mode waveguides with gradient index profiles, showing minimal modal dispersion.

The nanorods/nanowires/nanobullets formed at mild conditions show increased fracture toughness and absence of residual stress.

The fracture toughness of the nanorods/nanowires/nanobullets can be determined by force displacement measurements of cantilever/indentation three point bending tests in atomic force microscopy (AFM) and optical measurement of the indentation crack lengths. Elastic moduli can be determined by resonant frequency measurements.

The morphology and composition of the nanorods/nanowires/nanobullets can be analysed by transmission electron microscopy (TEM), scanning electron microscopy, (SEM), energy dispersive X-ray (EDX) and power diffractions (XRD) analysis, as well as Fourier transform infrared spectroscopy with attenuated total reflection (FTIR-ATR).

It is possible to incorporate luminescent or fluorescent molecules or fluorescently-labeled nanoparticles, e.g., Cy3-, Cy5-, or tris-(2,2'-bipyridyl)dichlororuthenium (II)-doped fluorescent silica nanoparticles, in the self-assembled nanorods/nanowires/nanobullets during their formation.

The propagation of locally excited fluorescence along the nanorods/nanowires/nanobullets can be determined using a pulsed laser source and a detector perpendicularly arranged to the waveguide axis. The locally excited fluorescence propagates towards and out-couples at the tip of the structures.

It is possible to fabricate low-loss optical waveguides with sub-wavelength diameters if the size of the silica nanoparticles is selected smaller than 10-20 nm. In addition, sol-gel chemistry methods can be applied to improve the homogeneity and surface smoothness of the nanorods/nanowires/nanobullets. Density fluctuations caused by the incorporated silica nanospheres may limit the range of application of the nanorods/nanowires/nanobullets to short distances (propagation loss caused by Rayleigh scattering due to surface roughness and local variations of the refractive index along the structures).

The advantages are:
a) The possibility to incorporate dopants at low temperature fabrication of the nanorods/nanowires/nanobullets allowing amplification of optical signals.
b) The low-temperature synthesis preventing the development of residual thermal stresses occurring as a result of cooling of the fibre, resulting in birefringence.

A further aspect of the invention is the generation of waveguides containing integrated recombinant poriferan luciferase (*S. domuncula*). Luciferases are enzymes that catalyze reactions that produce light in bioluminescence. The reaction is initiated by the formation of an enzyme-bound luciferyl adenylate from luciferin and ATP, catalyzed by the enzyme, followed by the conversion to an electronically excited-state product (under consumption of oxygen), which decays emitting a photon of visible light. During the flashing reaction luciferin is converted to oxyluciferin, a competitive inhibitor of luciferase.

The luciferase reaction displays a bioluminescence emission in the range of 490 nm to 620 nm. Integration of His-tag-luciferase can be achieved by immobilization of the enzyme on the surface of γ-Fe₂O₃ nanoparticles (via a reactive polymer), or by entrapment of luciferase in silica nanoparticles formed by silicatein. These nanoparticles can be incorporated in the nanorods/nanowires/nanobullets generated by addition of the scaffolding protein silintaphin-1 and silicatein. These particles emit light in the presence of luciferin and ATP in the surrounding medium.

The waveguiding properties of the nanorods/nanowires/nanobullets can be demonstrated by incorporation of luciferase-containing nanoparticles close to one tip of the structure and by incorporation of γ-Fe₂O₃ particles (coated by silicatein only) close to the opposite tip. The discrete incorporation of the nanoparticles can be achieved by addition of the nanoparticles to the reaction mixture after different time periods. The nanoparticles allow achievement of the appropriate orientation of the nanorods in a magnetic field. In the presence of substrate (ATP and luciferin) light is emitted and can be detected at the tips of the nanorods using an epifluorescence microscope, connected to an ultralow-light-level photon-counting camera.

The refractive index of the nanorods/nanowires/nanobullets can be determined by interferometry.

Luminescence resulting from the luciferase reaction can be measured after addition of luciferin, ATP, CoA, and MgCl₂ in a suitable reaction buffer: the light intensity can be detected using a luminometer whereas the emission spectrum can be determined spectrophotometrically.

A further aspect of this invention is the discovery that the tight packaging of the silica nanoparticles caused by the structure directing molecule silintaphin-1 unexpectedly facilitates a process usually known to occur only at high temperatures: sintering.

Sintering is widely used in ceramic production. In the sintering process, a ceramic powder is transformed into a bulk material by thermal treatment below the melting temperature of the main constituent material. Silica and many other metal oxides (zirconia, alumina, ferric oxide etc) sinter. The driving force in sintering is the reduction of surface energy through particle growth and shrinking of pores.

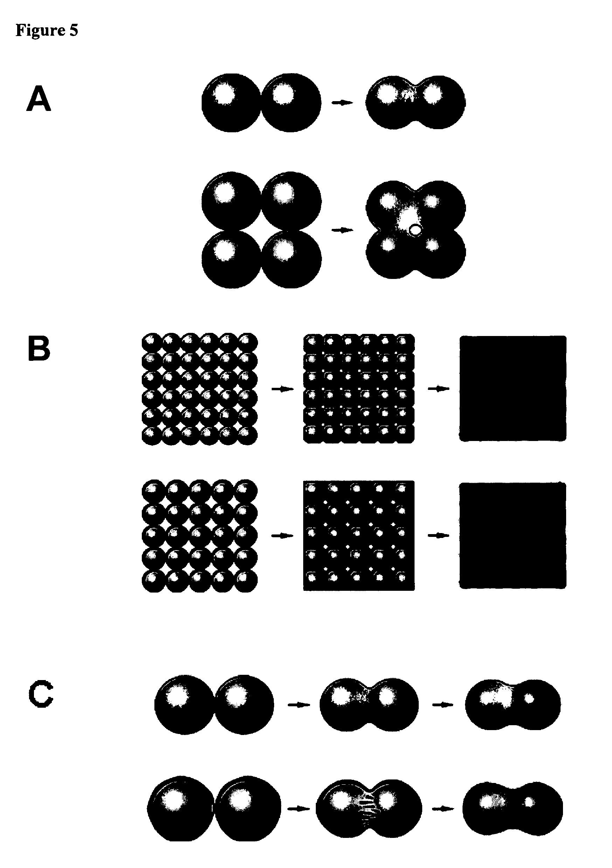

In the initial stage of sintering of spherical silica (nano)particles, circular necks (material bridges) are formed between the particles (Figure 5A, upper panel). As a result of neck formation and neck growth, the particles get closer to each other (shrinkage process) and the porosity decreases (Figure 5A, lower panel). Finally, the pores become closed (Figure 5B, upper panel). Mass transport during sintering occurs by diffusion mechanisms which strongly depend on temperature.

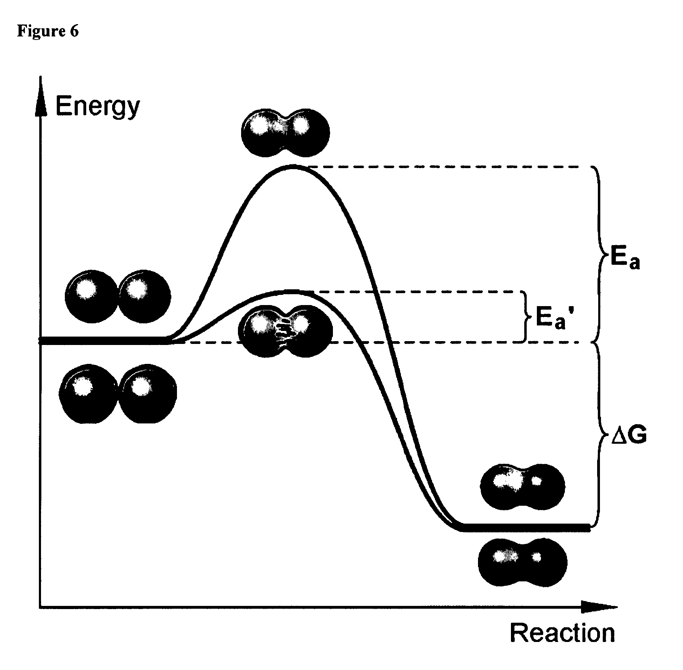

Sintering is a thermally activated process which usually needs high temperatures. The tight packaging of the silica nanoparticles (or nanoparticles of other metal oxides) in the nanorods/nanowires/nanobullets directed by silintaphin-1 alone or in cooperation with other molecules (silicatein or extracts of sponge spicules) allows this process to occur at temperatures which are at least two-times lower than the temperatures required for sintering according to state-of-the-art procedures. In spicules this process ("biosintering") was even observed at ambient temperature and pressure. Consequently, this process is characterized by a strong reduction in activation energy (Figure 6). The process of biosintering is novel and has not been observed before. This process is of high importance for many technical applications. It is assumed that the decrease in activation energy is caused (i) by promotion of the release of monomeric/oligomeric silicic acid molecules, used for neck formation, at the surface of the silica nanospheres through binding and depolymerisation / polymerization by silicatein, (ii) positioning and stabilization of silica nanosphere arrays by silinthaphin molecules facilitating the formation of stable silica bridges between adjacent silica nanospheres, and (iii) creation of a microenviroment, restricting the diffusion of silicic acid monomers to the inter-nanospheres space and possible creating optimal pH conditions for repolymerisation of silica for evolution of necks and fusion.

Biosintering can be further promoted by additional factors found in spicule extract.

### Application as optical fibre-based evanescent wave sensors

The fabrication of novel evanescent wave optical sensors, based on self-assembled, single-mode sub-wavelength-diameter nanorods/nanowires/nanobullets.

In principal, evanescent-field-based optical waveguide sensors can be used in absorbance and fluorescence modes. Fluorescent dyes can be bound to the surface of the nanorod/nanowire/nanobullet. In addition, antibodies can be immobilized to the functionalized structures formed by silicatein and silintaphin-1. Binding of fluorescent antigens recognized by the antibodies can be detected using a fluorescence microscope perpendicularly arranged to the waveguide axis.

The fraction of the light guided outside the nanorods/nanowires/nanobullets as evanescent waves is highly sensitive to changes of the index of the surrounding medium. Self-assembled, single-mode sub-wavelength-diameter nanorod/nanowire/nanobullet can be in a Mach-Zehnder-type interferometer to detect index changes caused by molecules interacting with the surface of the structures. The sensing part of the nanorod/nanowire/nanobullet is functionalized for the detection of the analyte in the aqueous solutions. The intensity and the optical phase of the guided light will change as a result of the index change after binding of the analyte. The Mach-Zehnder interferometer used to measure the phase shift of the light is assembled with two nanorods/nanowires/nanobullets, one of them sensing and exposed to the analyte, the other one the reference; an optical splitter (before the sensing area/reference area) and an optical combiner (after the sensing area/reference area) is formed by evanescent coupling of the two nanorods/nanowires/nanobullets, allowing measurement of the phase shift of the light by the interferometer.

Specific antibodies against selected proteins (for example tumour marker proteins) can be immobilized on the nanorods/nanowires/nanobullets surface as well as hydroxyapatite (HA) nanoparticles due to the HA-binding protein tag (Glu-tag) of recombinant silicatein or silintaphin-1.

### Application as optical-fibre based bacterial sensor

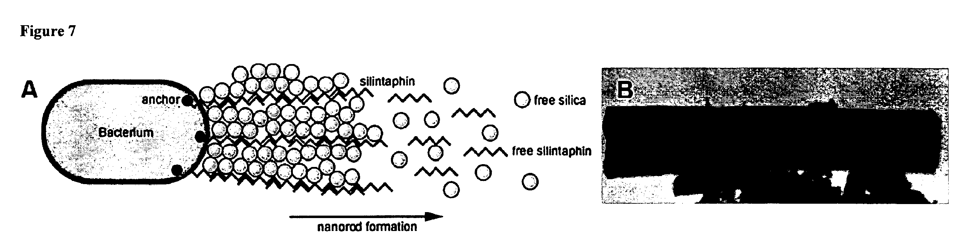

According to a further aspect of the invention, an optical-fibre based bacterial sensor can be prepared. Genetically engineered reporter strains of *E*. *coli* can be used in which a gene promoter (for example, a sensing element for genotoxic stress) is coupled to a reporter gene coding for a protein that generates a signal, e.g., fluorescence. These bacteria are furthermore transformed with an expression vector containing the silintaphin-1 cDNA linked to a sequence that codes for a bacterial cell wall anchor in combination with a protein translocation signal. Following expression and translocation the expressed chimeric protein is then partially extruded, thus allowing the assembly of silica particles (and silicatein) in the extracellular space (Figure 7A). In this way rod-like silica structures, which are intimately associated with the bacterium are formed, via a self-assembly mechanism (Figure 7B). The reaction conditions can be adapted so that silica rods with a diameter of >1 µm will be generated, which may act as multi-mode optical fibres (in a medium with lower refractive index). Generation of a layer of vertically arranged micro/nanorods on top of a monolayer of bacteria grown in a well plate allows the detection of the emitted light of individual bacteria, guided by the micro/nanorods after excitation by a perpendicular arranged light source.

According to a further aspect of the invention, the structures generated can be reinforced by encapsulation of the recombinant fluorescent bacteria in an optically transparent silica matrix. State-of-the-art methods developed by the inventors can be used for encapsulation of bacteria. This matrix can be formed either enzymatically (via silicatein) or via sol-gel techniques.

Genetically engineered bacteria obtained by fusion of the structural genes coding for green fluorescent protein (GFP) or red fluorescent protein (RFP) to the promoter of the recA gene of *E. coli* can be used. The expression of the fluorescent proteins by the recombinant *E. coli* strains in response to genotoxic stress is then determined.

According to a further aspect of the invention, these applications can be combined with other techniques, such as the encapsulation of bacterial sensors in a silica shell. We developed a technology for encapsulation of bacterial cells in a silica shell under mild conditions (absence of aggressive chemicals that may damage cells) (Müller et al. Biomaterials 29, 771-779, 2008). Bacteria are transformed with the silicatein gene. Silicatein expressed on the surface of the bacteria is then used for biocatalytic formation of the silica shell. The biosilica shell formed by silicatein does not impair the growth of the cells.

### Application of soft lithography (microfluidic) techniques

Alternatively, microfluidic lithography techniques (micromolding in capillaries) can be applied to obtain silica nanorods/nanowires/nanobullets with waveguiding properties, based on silintaphin-1-driven nanoparticle organization. The capillaries formed using this technique (nano-and microchannels) are filled, by capillary forces, with a solution containing recombinant silintaphin-1 (and silicatein). The generated surface pattern (after removal of the PDMS template) consisting of deposited silintaphin-1 molecules is then incubated with silica nanospheres (and TEOS) which will react with the surface bound silintaphin-1 (and silicatein) under formation of stable silica rods/wires/bullets.

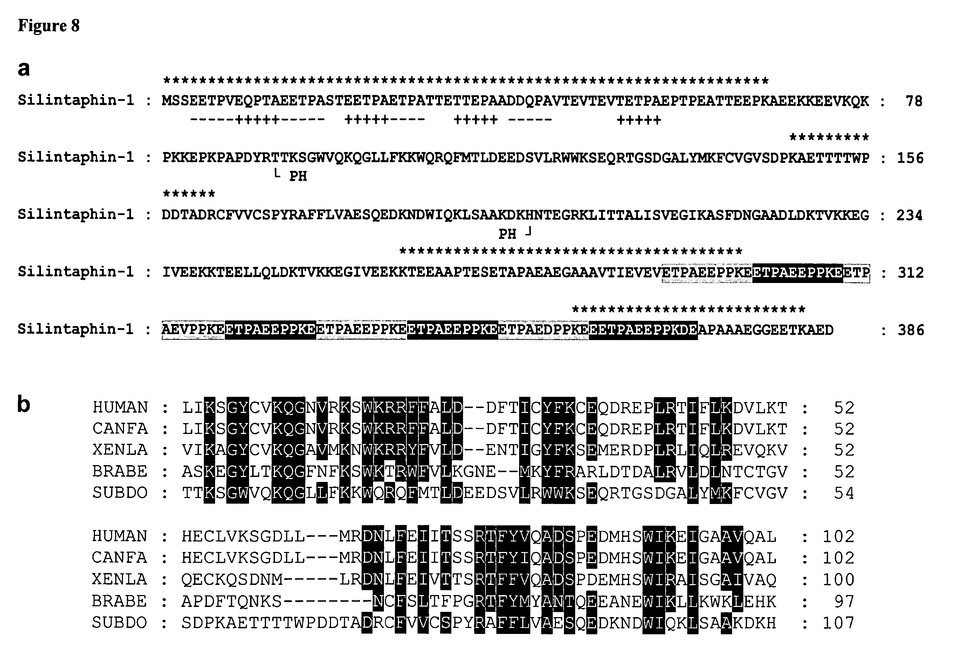

According to a further aspect of the invention, a procedure for the fabrication of nanorods/nanowires/nanobullets from metal oxide (nano)particles is presented, wherein a polypeptide is used for the synthesis, comprising an animal, bacterial, plant or fungal silintaphin-1 domain that exhibits a sequence similarity, preferably identity, of at least 25% to the sequence shown in SEQ ID No. 1 and Figure 8. Further preferred is a percent similarity of at least 50%, more preferred of at least 75%. Even more preferred is a percent identity of said domain of at least 50%, more preferred of at least 75%.

The metal oxide particles used for synthesis are coated with a silintaphin-1-interacting molecule, for example a metal oxide forming protein, such as silicatein, e.g., *Suberites domuncula* silicatein.

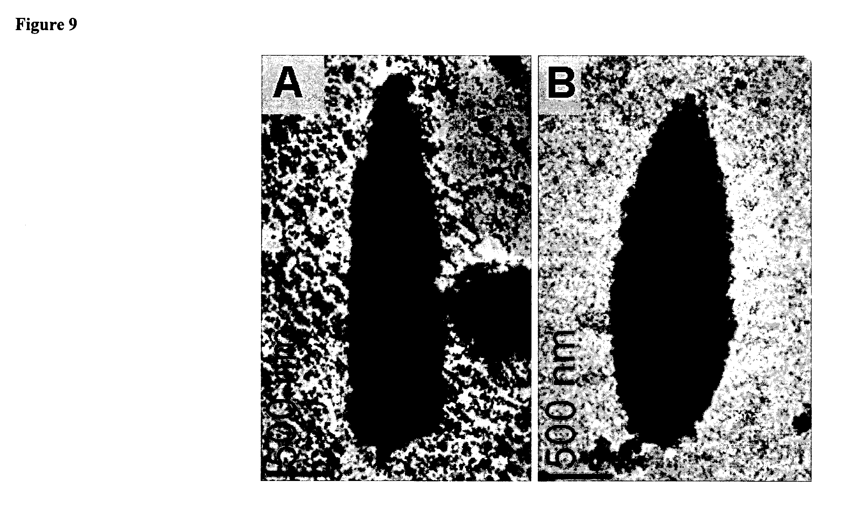

The metal oxide particles used for the fabrication of the nanorods/nanowires/nanobullets can consist of silica, titanium oxide, zirconium oxide or supermagnetic iron oxide (Figure 9).

It is also possible to use, instead of one type of metal oxide particles, a mixture of two or several metal oxides.

A further aspect of the invention concerns a chemical compound or silica-containing structure or surface which has been obtained by using the procedure described herein.

A further aspect of the invention concerns a fusion protein of *S*. *domuncula* silintaphin-1 (according to SEQ ID No. 1 and Figure 8) or a homologous polypeptide (silintaphin-1 domain) which has at least 25% amino acid sequence similarity, preferably identity, to the sequence shown in SEQ ID No. 1 and Figure 8, or parts thereof. Further preferred is a percent similarity of at least 50%, more preferred of at least 75%. Even more preferred is a percent identity of said domain of at least 50%, more preferred of at least 75%.

A further aspect of the invention concerns a nucleic acid, in particular according to SEQ ID No. 2, wherein this nucleic acid codes for a polypeptide described in this patent application. The nucleic acid can be a DNA, cDNA, RNA or a mixture thereof. The sequence of the nucleic acid can comprise at least one intron and/or a polyA sequence.

A further aspect of the invention concerns a nucleic acid in form of (a) a fusion protein (chimeric protein) construct or (b) construct with separate protein domains (separated by a protease cleavage site). The nucleic acid can also be produced synthetically. The necessary methods are state of the art.

A further aspect of the invention concerns a vector, preferentially in form of a plasmid, shuttle vector, phagemid, cosmid, expression vector, retroviral vector, adenoviral vector or particle, nanoparticle or liposome, wherein the vector contains a nucleic acid according to the invention. Furthermore, these vectors can be used for the transfer of proteins, preferentially in form of nanoparticles or liposomes, comprising a fusion protein according to the invention.

A further aspect of the invention is a host cell transfected with a vector or infected or transduced with a particle according to the invention. This host cell can express a polypeptide according to the invention or parts thereof. Any known host cell organism such as yeast, fungi, sponges, bacteria, mammalian or insect cell line can be used.

The fusion protein claimed herein can be produced synthetically or be present in a prokaryotic or eukaryotic cell extract or lysate. The cell extract or lysate can be prepared from a cell *ex vivo* or *ex vitro*, for example from a recombinant bacterial cell.

The fusion protein claimed herein can be purified using state-of-the-art methods and can therefore be essentially free of other proteins.

### Application as photonic crystals and photonic crystal fibres

The possibility to direct the assembly of silica nanoparticles by the combined action of silicatein and silintaphin-1, according to the invention, opens new synthetic strategies to obtain microstructured fibres (photonic crystal fibres), including photonic-bandgap fibres (hollow-core photonic crystal fibres). Moreover, structures combining this technology and the technique for encapsulation of bacterial cells (or biomolecules) provide novel sensors which can be used for a variety of technical applications.

### Application as light-emitting diodes (LEDs)

The incorporation of a photonic crystal, obtained according to the invention, into an LED can enhance the internal quantum efficiency and the amount of light extracted. Silicatein and silintaphin-1 can therefore be used for the formation of photonic crystal lattices for high-efficiency lighting applications.

Advantages of the technology according to the invention compared to conventional procedures are:
- The formation of nanorods/nanowires/nanobullets can be performed at mild conditions (low temperature, low pressure, near-neutral pH) (silicatein reaction)
- The reaction is controllable (enzymatic reaction)
- The reaction is structure-directed (silintaphin-1)
- The materials formed show a combination of unique properties: light transmission and extreme mechanical stability
- The silica synthesis does not involve the use of an alcohol (protein denaturant), or an alcohol released from some precursor (TEOS), like conventional technical procedures, for example the Stoeber method

### Expression and isolation of recombinant silintaphin-1

The preparation of the recombinant silintaphin-1 is preferentially performed in *E. coli*. However, expression of the recombinant protein in yeast and mammalian cells is also possible and has been successfully performed. The combination of an appropriate forward primer, for example: 5-TCATCAGAAGAGACCCCAGTAGA-3 (SEQ ID No. 3), and of an appropriate reverse primer, for example: 5-GTCTTCTGCTTTTGTCTCCTCA-3 (SEQ ID No. 4) is used to amplify the silintaphin-1 ORF (nt₁₀₇₋₁₂₆₁), excluding Mₛₜₐᵣₜ and stop codon. The amplicon is inserted into an expression vector, for example: pTrcHis2-TOPO (Invitrogen), in frame with an N-terminal Mₛₜₐᵣₜ and a C-terminal 6xHis-tag. Following transformation of BL21 cells the recombinant protein can be purified, e.g., by using the histidine-tag, on suitable affinity matrices, such as Ni-NTA (nickel-nitrilotriacetic acid affinity chromatography) under native conditions (Qiagen, Hilden, Germany). Protein concentrations can be measured with commercial kits, for example the "2-D Quant Kit" (GE Healthcare, München, Germany). Subsequently, the protein is analyzed by SDS-PAGE. After blotting on PVDF membranes (Millipore, Billerica, MA), the recombinant protein is detected with anti-histidine antibodies (Qiagen).

Alternatively, to confer hydroxyapatite (HA)-binding capacity to silintaphin-1, the recombinant protein can be bioengineered so that it obtains a novel protein-tag, developed by us. This novel tag comprises 8-10 glutamic acid residues (Glu), attached N-terminal or C-terminal to the protein and was derived from several mammalian proteins (e.g., osteonectin, bone sialoprotein) that have a strong binding affinity to (HA). Accordingly, the aforementioned primers are adapted, e.g., the forward primer sequence 5-GAAGAGGAAGAGGAAGAGGAAGAGTCATCAGAAGAGACCCCAGTAGA-3 (SEQ ID No. 5), carries 8 additional 5'-terminal triplets (underlined) in frame, each one coding for Glu. After transfer into an appropriate expression vector, the resulting construct is cloned in bacteria, yeast, or mammalian systems. Following induced expression the protein is purified as already described above.

### Isolation and purification of silintaphin-1 using antibodies

Silintaphin-1 can be further purified on an affinity matrix. The affinity matrix can be prepared, for example, by immobilization of a silintaphin-1-specific antibody on a solid phase (CNBr-activated Sepharose or another suitable carrier). Monoclonal or polyclonal antibodies against silintaphin-1 can be used, which are prepared following standard methods (Osterman (1984) Methods of Protein and Nucleic Acid Research Vol. 2; Springer-Verlag [Berlin]). Coupling of the antibody to the matrix is performed according to the instructions of the manufacturer. Elution of purified silintaphin-1 is performed by pH change or change in ionic strength. Also other affinity matrices can be used.

### Detection of silicatein activity and synthesis of silica

To determine the enzyme activity of recombinant silicatein an assay is used, which is based on measurement of polymerized and precipitated silica after hydrolysis and subsequent polymerization of TEOS.

Measurement of enzymatic activity of recombinant silicatein is usually performed as follows. The protein is dialyzed overnight against a buffer suitable for the enzymatic reaction, for example 50 mM MOPS, pH 6.8 [other buffers within a pH range of 4.5 to 10.5 are suitable, too].

Reaction mixtures are supplemented with TEOS solution (for example: 1 ml of 1 - 4.5 mM). Enzymatic reaction can be performed at room temperature. The silica product is collected by centrifugation (12 000 x g; 15 min; +4°C), washed with ethanol and air-dried. The pellet is then hydrolyzed in 1 M NaOH. The dissolved silicate is then quantitatively determined using a molybdate-based assay, e.g., the Silicon Assay (Merck).

The following substrates can be used: tetraalkoxysilanes, trialkoxysilanols, dialkoxysilanediols, monoalkoxysilanetriols, dialkoxysilanols, monoalkoxysilanediols, monoalkoxysilanols, alkyl-, aryl- or metallotrialkoxysilanes, alkyl-, aryl- or metallosilanols, alkyl-, aryl- or metallosilanediols, alkyl-, aryl- or metallosilanetriols, alkyl-, aryl- or metallomonoalkoxysilanediols, alkyl-, aryl- or metallodialkoxysilanols, or other metal oxide precursors (for example, alkoxy compounds of gallium, zirconium oxide or titanium oxide, titanium(IV) bis(ammonium-lactato) dihydroxide, or hexafluorozirconate). Also mixtures of these substrates are processed by the enzyme. Thus mixed polymers can also be produced.

### Scanning electron microscopy

Equimolar concentrations (7 nM) of silintaphin-1, prepared under non-denaturing conditions, and refolded silicatein are co-incubated in PBS (pH 7.5, 3 h). Following dialysis via MF membrane filters (Millipore), the protein solution is spotted on siliceous wafers that are subsequently dried. Formation of filamentous protein structures is assessed through a high resolution field emission scanning electron microscope.

### Transmission electron microscopy

Recombinant silicatein is immobilized on γ-Fe₂O₃ or other metal oxide nanoparticles that have been surface-functionalized with the reactive polymer poly(pentafluorophenyl acrylate) according to state-of-the-art techniques. In short, functionalized nanoparticles are treated successively with 1 mmol NaOH (30 min) and 40 mmol NiSO₄ (1 h). Subsequently, the Ni²⁺-binding particles are incubated with 40 mg/ml His-tagged silicatein in PBS (1 h), prior to the addition of silintaphin-1 (200 mg/ml PBS, 1 h). Ultimately, the samples are washed in PBS and analyzed using a transmission electron microscope.

### SDS-PAGE and Western blotting analyses

Proteins of the resulting supernatants are subjected to SDS-PAGE, transferred to PVDF membranes, and probed for with anti-silintaphin-1 antibodies. The resulting immunocomplexes are visualized by successive incubation with species-specific HRP-coupled secondary antibodies, and the "BM Chemiluminescence Western Blotting Substrate" (Roche Applied Science). In control experiments, antibodies are adsorbed to recombinant protein prior to their use.

Polyclonal antibodies against purified silintaphin-1 can be raised in female Balbc/An mice according to state-of-the-art procedures.

### Synthesis of calcitic microlenses

Calcite microlenses are prepared by CO₂ gas diffusion reaction with CaCl₂ (2.5 mM) and polystyrene sulfonate (PSS; 0.1%). The resulting mesocrystals display a truncated trigonal morphology, mimicking the eye microlenses of sea stars and trilobites that are made of hexagonal calcite oriented towards the only direction that is not birefringent and grants clear sight, the [001] direction. In addition, increased CaCl₂ concentration (5 mM) induces the formation of uniform seminconvex circular crystalline superstructures, where curving depends on the PSS concentration (0.1-0.01%). Samples are assessed by polarized light microscopy, scanning electron microscopy (SEM; coupled to EDX), high resolution transmission electron microscopy (HRTEM) with Fast Fourier transform (FFT) diffraction analysis, optical dark-field microscopy, and dynamic light scattering.

### Coupling of nanorods/nanowires/nanobullets to calcite microlenses

Glu-tagegd silintaphin-1 (240 µg/ml TBS, pH 7.5; 12 h) is anchored to CaCO₃ microlenses (50 mg) and then incubated with recombinant, refolded silicatein-α and a combination of sodium metasilicate (100 µM) and silica nanospheres (100 µg). Crosslinking of silintaphin-1 and silicatein results in the formation of protein filaments. Furthermore, the catalytic activity of immobilized silicatein contributes to creating a silica coating. Subsequently, protein-protein interactions and binding of silica nanoparticles is confirmed via immunodetection on Western blots, TEM, SEM, EDX, and FTIR-ATR. In addition, the catalytic formation of the silica coating is confirmed via surface plasmon resonance (SPR), EDX, and AFM. Ultimately, the focusing ability, angular selectivity, spectral transmission, and signal enhancement of the micro-optical device is confirmed. For this purpose a scanning confocal optical microscopy will be employed, complemented by a pulsed laser system with electro-optic crystal modulator, and a collimated white light source coupled to an optical spectrum analyzer.

### The legends to the Figures and sequence protocols are as follows.

**Figure 1****. A.** Scanning electron micrograph of isolated spicules from the marine sponge *Suberites domuncula*. **B**. Computer model of the silica-forming enzyme, silicatein-α. Amino acids of the catalytic triad are in red. Amino acids of the Ser clusters are in green. **C.** Co-localization of silintaphin-1 and silicatein-α in the central axial filament of a longitudinally sectioned sponge spicule. The tissue section was co-incubated (a) with anti-silintaphin-1 and (b) with anti-silicatein-α antibodies **(inset).** The immunocomplexes formed were visualized using Cy3 (red) and Alexa Fluor 488 (green; **inset)** labelled secondary antibodies (laser scanning microscopy). The cell nuclei were DAPI-stained (blue). Bar 6.7 mm.
**Figure 2****. A.** Partial view of a rod formed by assembly of functionalized γ-Fe₂O₃ nanoparticles through silicatein-α and silintaphin-1 interaction (scanning electron micrographs). Incubation of silintaphin-1 with silicatein-α, immobilized on the surface of functionalized γ-Fe₂O₃ particles, resulted in rod-like shapes. **B**. Higher magnification of the selected box in A to visualize the γ-Fe₂O₃ nanoparticles. **C.** Formation of filaments by equimolar amounts of silicatein-α and silintaphin-1 on a wafer surface (transmission electron micrograph). Bars 100 nm (A); 10 nm (B); 0.2 mm (C).
**Figure 3****.** Structure directing activity of silintaphin-1: formation of rod-like structures through silicatein-α and silintaphin-1 interaction, not possible by silicatein alone. **A.** Formation of amorphous aggregates of silicatein and functionalized γ-Fe₂O₃ nanoparticles. **B.** Formation of a rod by incubation of silintaphin-1 with silicatein-α, immobilized on the surface of the functionalized γ-Fe₂O₃ nanoparticles (scanning electron micrograph). The amorphous aggregates obtained with silicatein alone are several magnitudes smaller in size than the rod-like structures formed after addition of silintaphin-1. Bars 20 nm (A); 500 nm (B).
**Figure 4****. A.** Similarity in the construction of technical optical fibres and sponge spicules. Optical fibres are cylindrical dielectric waveguides that transmit light along their axis by total internal reflection. **A,B.** Concentric arrangement of the silica layers of the spicules; (scanning electron micrographs). C. Schematic representation of a technical optical fibre used in telecommunication. The fibre consists of a core surrounded by a cladding layer, as well as a protective outer coating (jacket).
**Figure 5****. A.** Sintering mechanism of two silica nanospheres. The neck formation between two adjacent silica spheres is shown. **B.** Sintering of silica nanospheres in the absence (upper panel) and presence (lower panel) of "biocatalytic" proteins ("biosintering"). The silica nanospheres are embedded in and intimately linked to organic material (silicatein and silintaphin) that create a "biocatalytic" microenvironment favoring mass transport and fusion (neck formation) between adjacent silica nanospheres. **C.** Sintering mechanism of two silica nanospheres in the absence (upper panel) and presence (lower panel) of "biocatalytic" proteins ("biosintering").
**Figure 6****.** Activation energy of sintering of silica nanoparticles (or nanoparticles of other metal oxides) in the absence and presence of biocatalyst. To initiate conventional sintering processes activation energy (Eₐ) is required since fusion of inorganic particles is exergonic (ΔG negative). During biosintering however the magnitude of Eₐ is reduced due to the presence of the proteins silicatein and/or silintaphin-1 (E_{a'}). This reduction facilitates and accelerates the fusion process at ambient temperature and allows the free energy (ΔG) for the sintering process to be released. It is outlined that the silica nanoparticles are surrounded by proteins silicatein and/or silintaphin-1 (in red).
**Figure 7****.** A. Formation of nanorods/wires tightly attached to a bacterium transformed with a silintaphin-1 cDNA carrying a transmembrane anchor sequence. The silintaphin molecules remain bound to the bacterial membrane and initiate the assembly of silica nanoparticles added to the medium and secreted silintaphin molecules. Silicatein bound to the nanoparticles (not shown) may catalyze further silica deposition. **B.** Silica nanorod formed; diameter: 1.5 µm.
**Figure 8****.** Deduced amino acid sequence of the silintaphin-1 from *Suberites domuncula* and alignment of the PH-domain (Pleckstrin homology-like domain) with other species (human = homo sapiens; canfa = Canis familiaris; xenla = Xenopus laevis; Brabe = Branchiostoma belcheri tsingtaunese; subdo = Suberites domuncula)
**Figure 9****. A.** Assembly of silica nanoparticles in the presence of silicatein-α and silintaphin-1 to distinct rod-like/spicular shapes. **B.** Incubation of silicatein-α and silintaphin-1 with titanium bis-(ammonium-lactato)-dihydroxide resulted in the synthesis of nanostructured biotitania that assembled to spicular structures. Scanning electron microscopy (SEM).

**SEQ ID No. 1:** Amino acid sequence of the silintaphin-1 polypeptide from *Suberites domuncula*.
**SEQ ID No. 2:** Nucleic acid sequence of the cDNA coding for the silintaphin-1 polypeptide from *Suberites domuncula*.
**SEQ ID No. 3 to SEQ ID No. 5:** Primers as used in the experiments above.
**SEQ ID No. 6** to **SEQ ID No. 9:** Amino acid sequences of the PH-domains as aligned in Figure 8.
**SEQ ID No. 10:** Amino acid sequence of the PH-domain for Entamoeba histolytica.

## Claims

1. A method for producing nanorods/nanowires/nanobullets from metal oxide particles or a mixture of two or several metal oxide particles, wherein a polypeptide is used for the synthesis, **characterized in that** the polypeptide comprises an animal, bacterial, plant or fungal silintaphin-1 domain that exhibits a sequence similarity, preferably identity, of at least 25% to the sequence shown in SEQ ID No. 1 and SEQ ID Nos. 6 to 10.

2. The method according to claim 1, wherein in addition to metal oxide particles silintaphin-1 and Glu-tagged silicatein-coated CaCO₃ (nano)particles, or silicatein and Glu-tagged silintaphin-1-coated CaCO₃ (nano)particles, or silicatein-coated silica nanoparticles and Glu-tagged silintaphin-1-coated CaCO₃ (nano)particles, or silicatein-coated nanoparticles of other metal oxides and Glu-tagged silintaphin-1-coated CaCO₃ (nano)particles are used for the fabrication of nanorods/nanowires/nanobullets, or wherein instead of CaCO₃ other metal- or alkaline-earth metal carbonates are used for generation of nanorods/nanowires/nanobullets.

3. The method according to claim 1, **characterized in that** the metal oxide particles used for synthesis are coated with a silintaphin-1-interacting molecule.

4. The method according to claim 1, **characterized in that** the metal oxide particles used for the fabrication of the nanorods/nanowires/nanobullets consist of silica, titanium oxide, zirconium oxide, or supermagnetic iron oxide.

5. The method according to any of claims 1 to 3, wherein the silintaphin-1-interacting molecule is selected from a metal oxide forming protein, silicatein, and preferably silicatein from the sponge *Suberites domuncula*.

6. The method according to any of claims 1 to 5, wherein a silintaphin-1 polypeptide from *Suberites domuncula* according to SEQ ID No. 1 is used, or a polypeptide being homologous thereto, which in the amino acid sequence of the silintaphin-1 domain exhibits a sequence similarity of at least 25% to the sequence shown in SEQ ID No. 1, or parts thereof.

7. The method according to any of claims 1 to 6, wherein a silintaphin-1 polypeptide from *Suberites domuncula* according to SEQ ID No. 1 is used, or a polypeptide being homologous thereto, which in the amino acid sequence of the silintaphin-1 domain exhibits a sequence similarity of at least 25% to the sequence shown in SEQ ID No. 1, is provided *in vivo*, in a cellular extract or lysate or in purified form.

8. The method according to any of claims 1 to 7, wherein silicic acid, silicates, monoalkoxysilanetriols, monoalkoxysilanediols, monoalkoxysilanols, dialkoxysilane-diols, dialkoxysilanols, trialkoxysilanols, tetraalkoxysilanes, alkyl-, aryl- or metallo-silanetriols, alkyl-, aryl- or metallo-silanediols, alkyl-, aryl- or metallo-silanols, alkyl-, aryl- or metallomonoalkoxysilanediols, alkyl-, aryl- or metallo-monoalkoxysilanols, alkyl-, aryl- or metallodialkoxysilanols, alkyl-, aryl- or metallo-trialkoxysilanes or other metal oxide precursor compounds are used as substrates for synthesis.

9. The method according to any of claims 1 to 8, wherein mixed polymers of defined composition are produced using defined mixtures of said substrates.

10. The method according to any of claims 1 to 9, wherein further comprising facilitating densification of the material ("biosintering") by fusion of the silica particles or metal oxide particles, and/or further coupling said nanorods/nanowires/nanobullets to calcite microlenses (mimicking calcite microlenses of brittlestars and trilobites).

11. A nanorod/nanowire/nanobullet, produced according to a method of any of the preceding claims.

12. Polypeptide of a silintaphin-1 or a polypeptide being homologous thereto, or a nucleic acid according to SEQ ID No. 2, or a nucleic acid being homologous thereto, **characterized in that** it essentially or exclusively encodes for a polypeptide, which in the amino acid sequence of the silintaphin-1 exhibits a sequence similarity, preferably identity, of at least 25% to the sequence shown in SEQ ID No. 1, or functional parts thereof.

13. Nanocomposite material produced according to a method according to any of claims 1 to 10, optionally together with suitable additives and supplements.

14. Use of the nanorods/nanowires produced according to any of claims 1 to 11 as optical waveguides, as optical fibre-based evanescent wave sensors, as optical-fibre based bacterial sensor, as photonic crystals, as photonic crystal fibres, and/or as light-emitting diodes (LEDs).

15. Use according to claim 14 as optical waveguides, wherein specific antibodies against selected molecules are immobilized on the nanorods/nanowires/nanobullets surface as well as hydroxyapatite (HA) nanoparticles or nanoparticles consisting of CaCO₃ or other metal- or alkaline-earth metal carbonates, due to the Glu-tag of recombinant silicatein or silintaphin-1.


SEQUENCE LISTING

<110> Mueller, Werner
      Wang, Xiaohong
      Wiens, Matthias
      Schröder, Heinz

<120> USE OF SILINTAPHIN FOR THE STRUCTURE-DIRECTED FABRICATION OF
      (NANO)COMPOSITE MATERIALS IN MEDICINE AND (NANO)TECHNOLOGY

<130> FB21462

<160> 10

<170> PatentIn version 3.4

<210> 1
<211> 386
<212> PRT
<213> Suberites domuncula

<400> 1

Met Ser Ser Glu Glu Thr Pro Val Glu Gln Pro Thr Ala Glu Glu Thr
1               5                   10                  15

Pro Ala Ser Thr Glu Glu Thr Pro Ala Glu Thr Pro Ala Thr Thr Glu
            20                  25                  30

Thr Thr Glu Pro Ala Ala Asp Asp Gln Pro Ala Val Thr Glu Val Thr
        35                  40                  45

Glu Val Thr Glu Thr Pro Ala Glu Pro Thr Pro Glu Ala Thr Thr Glu
    50                  55                  60

Glu Pro Lys Ala Glu Glu Lys Lys Glu Glu Val Lys Gln Lys Pro Lys
65                  70                  75                  80

Lys Glu Pro Lys Pro Ala Pro Asp Tyr Arg Thr Thr Lys Ser Gly Trp
                85                  90                  95

Val Gln Lys Gln Gly Leu Leu Phe Lys Lys Trp Gln Arg Gln Phe Met
            100                 105                 110

Thr Leu Asp Glu Glu Asp Ser Val Leu Arg Trp Trp Lys Ser Glu Gln
        115                 120                 125

Arg Thr Gly Ser Asp Gly Ala Leu Tyr Met Lys Phe Cys Val Gly Val
    130                 135                 140

Ser Asp Pro Lys Ala Glu Thr Thr Thr Thr Trp Pro Asp Asp Thr Ala
145                 150                 155                 160

Asp Arg Cys Phe Val Val Cys Ser Pro Tyr Arg Ala Phe Phe Leu Val
                165                 170                 175

Ala Glu Ser Gln Glu Asp Lys Asn Asp Trp Ile Gln Lys Leu Ser Ala
            180                 185                 190

Ala Lys Asp Lys His Asn Thr Glu Gly Arg Lys Leu Ile Thr Thr Ala
        195                 200                 205

Leu Ile Ser Val Glu Gly Ile Lys Ala Ser Phe Asp Asn Gly Ala Ala
    210                 215                 220

Asp Leu Asp Lys Thr Val Lys Lys Glu Gly Ile Val Glu Glu Lys Lys
225                 230                 235                 240

Thr Glu Glu Leu Leu Gln Leu Asp Lys Thr Val Lys Lys Glu Gly Ile
                245                 250                 255

Val Glu Glu Lys Lys Thr Glu Glu Ala Ala Pro Thr Glu Ser Glu Thr
            260                 265                 270

Ala Pro Ala Glu Ala Glu Gly Ala Ala Ala Val Thr Ile Glu Val Glu
        275                 280                 285

Val Glu Thr Pro Ala Glu Glu Pro Pro Lys Glu Glu Thr Pro Ala Glu
    290                 295                 300

Glu Pro Pro Lys Glu Glu Thr Pro Ala Glu Val Pro Pro Lys Glu Glu
305                 310                 315                 320

Thr Pro Ala Glu Glu Pro Pro Lys Glu Glu Thr Pro Ala Glu Glu Pro
                325                 330                 335

Pro Lys Glu Glu Thr Pro Ala Glu Glu Pro Pro Lys Glu Glu Thr Pro
            340                 345                 350

Ala Glu Asp Pro Pro Lys Glu Glu Glu Thr Pro Ala Glu Glu Pro Pro
        355                 360                 365

Lys Asp Glu Ala Pro Ala Ala Ala Glu Gly Gly Glu Glu Thr Lys Ala
    370                 375                 380

Glu Asp
385

<210>  2
<211>  1611
<212>  DNA

<213>  Suberites domuncula

<400>  2
gggggatagg ggaaaattca tttctgcaga gttcagtgca gtaaagaaaa gaaggctaag      60

atatccagca tctcgaaaac tatctatctc aagcacttac accatgtcat cagaagagac     120

cccagtagaa cagcccacag ctgaggagac gccagcctct acagaggaga caccagcaga     180

gaccccagct accactgaaa caacagagcc agctgctgat gaccagcctg ctgtcacaga     240

ggtcacagag gtcactgaga ctccagccga gcctactcca gaagccacca cagaggagcc     300

caaggctgaa gagaagaaag aagaagtcaa acagaagccc aagaaggaac ccaagcctgc     360

cccagactac agaacaacaa agagtggctg ggttcagaaa caaggactgc tcttcaagaa     420

gtggcagcga cagttcatga ccctggatga ggaggatagt gtactaagat ggtggaagtc     480

tgaacaaagg actggctctg atggcgctct ctatatgaag ttctgtgttg gtgtgtctga     540

ccccaaggct gaaacaacaa ccacttggcc tgatgacaca gctgatcgat gctttgttgt     600

gtgctctcct tacagagcat tcttcctagt agcagagagt caagaggata agaatgactg     660

gatccagaaa ctttctgctg ccaaagataa gcacaacact gaaggccgta agctgatcac     720

tactgccctg atctctgtgg aaggcatcaa ggcaagtttt gataatggag ctgctgacct     780

ggacaagact gtcaagaaag agggcattgt tgaggagaag aagacagagg agctgctcca     840

actggacaag actgtcaaga aagagggcat tgttgaggag aagaagacag aggaggctgc     900

tccaactgag agtgaaacag ctcctgcaga ggcagaaggt gctgctgcag ttactataga     960

agttgaggtt gaaacaccag ctgaagagcc ccctaaagaa gaaactccag cagaggagcc    1020

acccaaagag gagacacccg cagaggttcc ccccaaagag gagacacccg cagaggaacc    1080

ccccaaagag gaaacacctg ctgaggagcc ccccaaagag gagacacccg cagaagaacc    1140

ccccaaagag gagacgcctg cagaggatcc ccctaaagaa gaagaaacac cagctgagga    1200

accaccaaag gatgaagcac cagcagcagc tgaaggaggt gaggagacaa aagcagaaga    1260

ctaaataaac tgtagcacac gataggatta ttattgtgga caaatactgt ttaaaaattg    1320

acgctatcag atcaaaagac ttacacgcac ataattatta ccttccacta cacttattag    1380

tttgttacat gtacaaatag atgcacattt ttgtattgta cactttttat acaaaggttt    1440

gtgttgttat tcctgtatgt gtatgatatg gatttttgtt tattctatag tacatggtcc    1500

ccttaattct gatttagaaa cttttctgta cattgagcag ttttactcca cttttagtgt    1560

gctgtgtgta atattatcaa attttgaata ataaatgttt tttgctgcaa a             1611

<210>  3
<211>  23
<212>  DNA
<213>  Suberites domuncula

<400> 3
tcatcagaag agaccccagt aga 23

<210> 4
<211> 22
<212> DNA
<213> Suberites domuncula

<400> 4
gtcttctgct tttgtctcct ca 22

<210> 5
<211> 47
<212> DNA
<213> Suberites domuncula

<400> 5
gaagaggaag aggaagagga agagtcatca gaagagaccc cagtaga 47

<210> 6
<211> 102
<212> PRT
<213> Homo sapiens

<400> 6

Leu Ile Lys Ser Gly Tyr Cys Val Lys Gln Gly Asn Val Arg Lys Ser
1               5                   10                  15

Trp Lys Arg Arg Phe Phe Ala Leu Asp Asp Phe Thr Ile Cys Tyr Phe
            20                  25                  30

Lys Cys Glu Gln Asp Arg Glu Pro Leu Arg Thr Ile Phe Leu Lys Asp
        35                  40                  45

Val Leu Lys Thr His Glu Cys Leu Val Lys Ser Gly Asp Leu Leu Met
    50                  55                  60

Arg Asp Asn Leu Phe Glu Ile Ile Thr Ser Ser Arg Thr Phe Tyr Val
65                  70                  75                  80

Gln Ala Asp Ser Pro Glu Asp Met His Ser Trp Ile Lys Glu Ile Gly
                85                  90                  95

Ala Ala Val Gln Ala Leu
            100

<210> 7
<211> 101
<212> PRT
<213> Canis familiares

<400> 7

Ile Lys Ser Gly Tyr Cys Val Lys Gln Gly Asn Val Arg Lys Ser Trp
1               5                   10                  15

Lys Arg Arg Phe Phe Ala Leu Asp Asp Phe Thr Ile Cys Tyr Phe Lys
            20                  25                  30

Cys Glu Gln Asp Arg Glu Pro Leu Arg Thr Ile Phe Leu Lys Asp Val
        35                  40                  45

Leu Lys Thr His Glu Cys Leu Val Lys Ser Gly Asp Leu Leu Met Arg
    50                  55                  60

Asp Asn Leu Phe Glu Ile Ile Thr Ser Ser Arg Thr Phe Tyr Ile Gln
65                  70                  75                  80

Ala Asp Ser Pro Glu Asp Met His Ser Trp Ile Lys Glu Ile Gly Ala
                85                  90                  95

Ala Val Gln Ala Leu
            100

<210>  8
<211>  100
<212>  PRT
<213>  Xenopus laevis

<400>  8

Val Ile Lys Ala Gly Tyr Cys Val Lys Gln Gly Ala Val Met Lys Asn
1               5                   10                  15

Trp Lys Arg Arg Tyr Phe Val Leu Asp Glu Asn Thr Ile Gly Tyr Phe
            20                  25                  30

Lys Ser Glu Met Asp Arg Asp Pro Leu Arg Val Ile Gln Leu Arg Glu
        35                  40                  45

Val Gln Lys Val Gln Glu Cys Lys Gln Ser Asp Asn Met Leu Arg Asp
    50                  55                  60

Asn Leu Leu Glu Ile Val Thr Thr Phe Arg Thr Phe Phe Val Gln Ala
65                  70                  75                  80

Asp Ser Pro Asp Glu Met His Ser Trp Ile Arg Ala Ile Ser Gly Ala
                85                  90                  95

Ile Val Ala Gln
            100

<210>  9
<211>  97
<212>  PRT
<213>  Branchiostoma belcheri

<400>  9

Ala Ser Lys Glu Gly Tyr Leu Thr Lys Gln Gly Phe Asn Phe Lys Ser
1               5                   10                  15

Trp Lys Thr Arg Trp Phe Val Leu Lys Gly Asn Glu Met Lys Tyr Phe
            20                  25                  30

Arg Ala Arg Leu Asp Thr Asp Ala Leu Arg Val Leu Asp Leu Asn Thr
        35                  40                  45

Cys Thr Gly Val Ala Pro Asp Phe Thr Gln Asn Lys Ser Asn Cys Phe
    50                  55                  60

Ser Leu Thr Phe Pro Gly Arg Thr Phe Tyr Met Tyr Ala Asn Thr Gln
65                  70                  75                  80

Glu Glu Ala Asn Glu Trp Ile Lys Leu Leu Lys Trp Lys Leu Glu His
                85                  90                  95

Lys

<210>  10
<211>  91
<212>  PRT
<213>  Entamoeba dispar

<400>  10

Ser Val Lys Ser Gly Phe Leu Val Lys Gln Gly Gly Ser Trp Lys Ser
1               5                   10                  15

Trp Lys Lys Arg Trp Cys Val Leu Thr Pro Thr Gly Met Ile Phe Tyr
            20                  25                  30

Phe Lys Asp Lys Lys Asp Val Asn Ser Lys Gly Cys Val Asp Val Asn
        35                  40                  45

Ser Ala Ser Asp Val Leu Leu Glu Asp Glu Lys Lys Lys Asn Cys Phe
    50                  55                  60

Gly Ile Val Thr Pro Asn Arg Thr Phe Phe Met Ala Ala Glu Ser Lys
65                  70                  75                  80

Ala Glu Arg Asp Ser Trp Ile Gln Ala Val Ser
                85                  90